# EUROPEAN PATENT APPLICATION

(11) **EP 3 100 669 A1**
(43) Date of publication of application: **07.12.2016**
(21) Application number: 15170819.5
(22) Date of filing: 05.06.2015
(51) Int. Cl.: A61B 1/06, A61B 1/07

(54) **A SPECTROSCOPIC IMAGING DEVICE**

(71) Applicant: University of Limerick, Limerick (IE)
(72) Inventor: The designation of the inventor has not yet been filed
(74) Representative: Lucey, Michael

(57) **Abstract**

The present invention provides an endoscopic mid infrared imaging device. The device comprises a mid infrared, MIR, light source; at least one optical fibre adapted to transport MIR light emitted from the MIR light source; an imaging probe coupled to the optical fibre, wherein the imaging probe is adapted to provide a MIR light beam to an imaging area; and optics adapted to control the size of the MIR light beam for imaging.

## Description

### Field of the Invention

The present invention is concerned with how to provide real time chemical imaging of disease causing molecular markers in a living system.

### Background to the Invention

One technique used to detect and determine the onset and the various stages of a pathological state that may later turn into a life-threatening disease is through the mapping of molecular signatures in biomarkers. Currently the only way to provide such information with certainty is via off site biopsy, through a histochemical and an immunohistochemical assay analysis of an extracted sample, which can take between 3 and 4 days. Optical biopsy involves the taking of optical images of tissue and cellular sections on site. It uses fluorescent staining or phase contrast imaging to enable local visualisation. Disease identification is then carried out through online pattern recognition using a library of comparisons between optical images of healthy and pathogenic samples. While optical biopsy does not eliminate taking the biopsy samples, it provides assistance in obtaining more targeted biopsy samples.

The possibility of direct mapping of the chemistry of biomarkers, that is without the need to stain, has been assessed in the diagnosis of various tissue diseases, such as lung cancers, breast cancer, prostate cancer, peritonea, brain cancer, skin cancer, as well as other tissue disorders, such as liver steatosis, heart disorder, and radiation induced damages into cells. This direct mapping has been explored using different infrared (IR) spectroscopic imaging techniques, such as an IR microscope equipped with a thermal IR source, an IR microscope equipped with a thermal IR source and focal plane array detector (FPA) and an IR microscope fitted to a synchrotron beam line. The image acquisition time for an IR microscope that uses a multiple beam synchrotron IR source is 30 minutes, when compared to IR acquisition time for a single beam being approximately 11 hours. US Patent No. 6,442,419 discloses IR thermal imaging of both the shape and size of external and internal body parts. US Patent No. 6,501,551 discloses a fibre optic based optical coherence tomography (OCT) system for endoscopic detection. While both of these IR imaging systems are capable of producing morphological images, they are incapable of revealing chemical constituents.

It is also known to perform minimally invasive surgical procedures for accessing internal organs and tissues. A number of devices currently exist that reach different body parts through such procedures which usually are conducted through one or more forms of in vivo imaging. For example, drug coated intravascular stents are deployed in treating coronary artery diseases under the guidance and follow-up assessment using techniques such as intravascular ultrasonography (IVUS). However IVUS does not provide any chemical information, the achievable resolution and assessment capabilities are very limited, and it requires acoustic coupling that further limits its efficacy for image guided intervention, such as for the staging of tumour growth progression.

It is also known to provide imaging via an endoscope. One known fibre endoscope comprises a flexible optical fibre cable composed of one or more glass or plastic fibres for transferring images from the distal end to the proximal end of the endoscope. However, such light based endoscopes do not offer any depth information. Techniques such as fluorescence, confocal, and multiphoton microscopy have been used to obtain depth information. However, for endoscopic applications, the primary design consideration is the size of the impinging distal probe that will work as an objective and the size of the transmitting optical fibre that will provide the conduit of the light that will be used for imaging. Both single-mode and multimode fibres may be used. In many cases, a gradient-index fiber can be employed to re-focus the diverging light from the delivery fiber at the distal end. Spectroscopic data may also be obtained from such techniques, in order to enable a multidimensional view of the sample under investigation.

Fiber-based visible/Near IR (NIR) light endoscopic probes may be used in both forward-viewing and side-viewing configurations, with the majority of endoscopic probes being of the side-viewing variety. Side-imaging endoscopes are suitable for imaging within a tubular organ such as the vascular system, gastro intestinal and urinary tracts. In contrast to side-imaging endoscopy, forward-imaging endoscopy can be useful in providing tissue structural information for surgical guidance or device placement. For *in vivo* 3-D imaging, either circumferential or linear scanning can be used in conjunction with a proximal or distal scanning mechanism. Current visible light endoscopy allows fast scanning. For example, a miniature motorised endoscopic probe capable of 200-1200 frame per second (fps) has been reported. In addition, polarisation and dispersion mismatch issues, and an increase in the vibration tolerance of such systems can be circumvented through common-path approaches. Recent work has been reported outlining the development of a single fibre endoscope as thin as a human hair, representing a 4-fold resolution enhancement by an input of sequences of random field patterns. These fibres have been developed for imaging with ultraviolet (uv)-visible- and NIR (up to 2.5 micron wavelength) radiation.

It is known that mid- infra-red (MIR) radiation (2.5 - 20 micron wavelength) is capable of revealing molecular chemistry. When a molecular solid, liquid or gas is excited by a radiation in the infrared spectrum, the molecule produces a vibrational spectra in the MIR wavelength region (2.5 -20 micron or 4000 -500 cm⁻¹), known as its molecular fingerprint. The molecular fingerprint reveals its molecular constitutions, bonding and characteristic molecular structure. It is known to assign various regions of IR spectra that arise from different types of generic molecular organisation and specific 'finger print' regions of such molecular structures. This is particularly beneficial for tissue, cellular and proteins in identifying their 'pathological' states which are different from their 'normal' states in molecular signatures (i.e. their biochemistry). The zone 500-900 cm⁻¹ has also been identified as the region of molecular finger printing for nuclei constituents such as DNA, RNA, and minerals. Proteins, for example, are well characterised in the region 1500-1800 cm⁻¹ as well as 2850-2950⁻¹. Chalcogenide optical fibres also exist that are capable of transmitting MIR. However, the typical cut off of these optical fibres is around 10 micron. Silver halide based MIR optic fibres are also capable of transmitting MIR in the range of 2-18 micron. US Patent No. 7,956,317 discloses some of these probes for M I R spectroscopy.

Molecular imaging of living cells/tissues using MIR has not been successful to date. This is due to a number of issues. Due to the low thermal intensity and broadband nature of current MIR sources, MIR imaging offers very low resolution, which is of the order of 10-70 micron *in vitro.* The acquisition time for such imaging is excessively high and impractical for *in vivo* imaging due to the possibility of tissue damage but also the requirement of the patient to be under investigation for such a long time. There is also technical difficulties around the transportation of MIR excitation radiation and image signals, especially to internal organs.

In standard IR microscopy, a full IR spectrum is obtained at each pixel, which is then averaged. Scanning an area of 5x5 mm² can take several tens of minutes even with a larger beam spot size (∼50-70 µm) and pixel size (e.g. 50 µm). For practical endoscopy, fast scanning of a large area of tissue at a reasonable spatial and pixel resolution is critical for identifying suspect areas of pathology development. However, improving the spatial resolution to a few µm alongside a pixel size that enhances spectral contrast and approaches current standard video quality leads to impractical image acquisition time using current MIR imaging sources. For example, imaging a 280x 310 µm² tissue area using a synchrotron source and a dual aperture IR microscope and diffraction limited resolution would require 11 days.

Further restrictions to the use of IR imaging for *in vivo* diagnostics come from the damage threshold and access to the diseased sites, the size of the anatomical site, and the speed of recognition of pathology with respect to healthy tissue, cells or biomolecule.

Therefore, there exists a need to provide a system and method to provide in vivo imaging which overcomes at least one of the above mentioned problems.

### Summary of the Invention

According to the invention, there is provided, as set out in the appended claims, an endoscopic mid infrared in vivo imaging device comprising:
a mid infrared, MIR, light source;
at least one optical fibre adapted to transport MIR light emitted from the MIR light source;
an imaging probe coupled to the optical fibre, wherein the imaging probe is adapted to provide a MIR light beam to an imaging area; and
optics adapted to control the size of the MIR light beam for imaging.

The MIR light source may comprise a laser integrated with a matching optical parametric oscillator, OPO, wherein the laser is adapted to generate picosecond infrared pulses at a high repetition rate with a tuneable wavelength.

The pulse repetition rate may be greater than 30 MHz.

Preferably, the pulse repetition rate is greater than 100 MHz.

The wavelength of the laser may be tuneable between 2.5 and 20 microns.

The size of the MIR light beam may be equivalent to or lower than the wavelength of the MIR light source.

The device may comprise a proximal end and a distal end, wherein the MIR light source is provided at the proximal end, and wherein the optics are provided at both the distal end and at the proximal end.

The at least one optical fibre may comprise one optical fibre adapted to transport MIR light emitted from the MIR light source from the proximal end to the distal end and one or more other optical fibres.

The one or more other optical fibres may be adapted to transport near infrared or visible light.

The one or more other optical fibres may be adapted to transport images collected from the imaging area from the distal end to the proximal end of the device.

The at least one optical fibre may comprise polycrystalline silver halide optical fibres.

The optics may be adapted to control the size of the MIR light beam to provide large area imaging at the video rate or higher.

The optics may comprise a lens or zooming optics at the distal end of the device and a relay lens at the proximal end of the device.

The optics may comprise a CaF2 or ZnS or gradient index lens or zooming optics at the distal end of the device.

The optics at the distal end of the device may be adapted to provide high spatial resolution imaging.

The high spatial resolution may comprise sub wavelength resolution or super resolution.

The super resolution may be provided by the optics at the distal end of the device adapted to generate a doughnut optical pattern.

A phase plate may be adapted to generate the optical pattern.

A circular mirror with a circular deformation in its centre may be adapted to generate the optical pattern.

A helicoidal deformable mirror may be adapted to generate the optical pattern,

The optics may be adapted to operate in an attenuated total reflection, ATR, configuration.

The imaging probe may comprise a Si hemisphere.

The ATR configuration may comprise a photoinduced aperture scheme combined with refringent hemisphere for super resolution microscopy.

The imaging probe for the photoinduced aperture scheme may comprise a silicon/geranium film plate deposited on the flat fact of a ZnS or ZnSe hemisphere.

The at least one optical fibre may be adapted to transmit a Gaussian MIR beam and a visible light doughnut beam.

The device may further comprise one optical fibre adapted to transmit the Gaussian MIR beam and one optical fibre adapted to transmit the visible light doughnut beam.

The optics at the distal end of the device may be adapted to provide diffraction limited resolution imaging.

The optical fibre may comprise a single mode optical fibre.

The optics may further comprise an up-conversion material for upconverting the images collected from the imaging area into visible or NIR.

The up-conversion material may comprise a multi-channel plate.

The wavelength output from the OPO may be adapted to be modulated around a wavelength of interest, and wherein the MIR light for imaging may comprise the second harmonic of the MIR spectra.

The device may further comprise a detection system adapted to detect imaging signals in the MIR, NIR and visible light range.

The detection system may comprise an electron magnified camera or a CCD camera.

The device may further comprise a scanner adapted to scan the MIR light beam or scan the imaging probe to provide point-to-point spectroscopic imaging.

The scanner may comprise a nanoscanner piezoelectric system.

The device may further comprise a suction system adapted to attach the imaging probe to the imaging area.

### Brief Description of the Drawings

The invention will be more clearly understood from the following description of an embodiment thereof, given by way of example only, with reference to the accompanying drawings, in which:-
Figure 1 shows the basic principle of MIR imaging using the device;
Figure 2 shows the imaging speed of the MIR device imaging using a 200 MHz pulse MIR laser;
Figure 3 shows a number of configurations of MIR Imaging provided by the device of the present invention;
Figure 4 illustrates the creation of doughnut optical pattern by the device using a deformable mirror;
Figure 5 illustrates the creation of doughnut optical pattern by the device using a helicoidal mirror;
Figure 6 shows a diagram of the device using a photoinduced aperture scheme in the MIR optical fibre to achieve super-resolution;
Figure 7 shows a diagram of a the device with a single mode MIR optical fibre for diffraction limited resolution imaging; and
Figure 8 shows graphs of simulated spectral dispersion for a wavelength range of 0.4 - 20 µm for (a) fused silica (b) SiO2 doped with 13.5% GeO2 and (c) AgBr fibers, each employing a FWHM spectral pulse linewidth of 1.6 µm over a fiber propagating length of 1 m.

### Detailed Description of the Drawings

The present invention provides for the real time chemical imaging of disease causing molecular markers in a living system through the use of endoscopic MIR imaging.

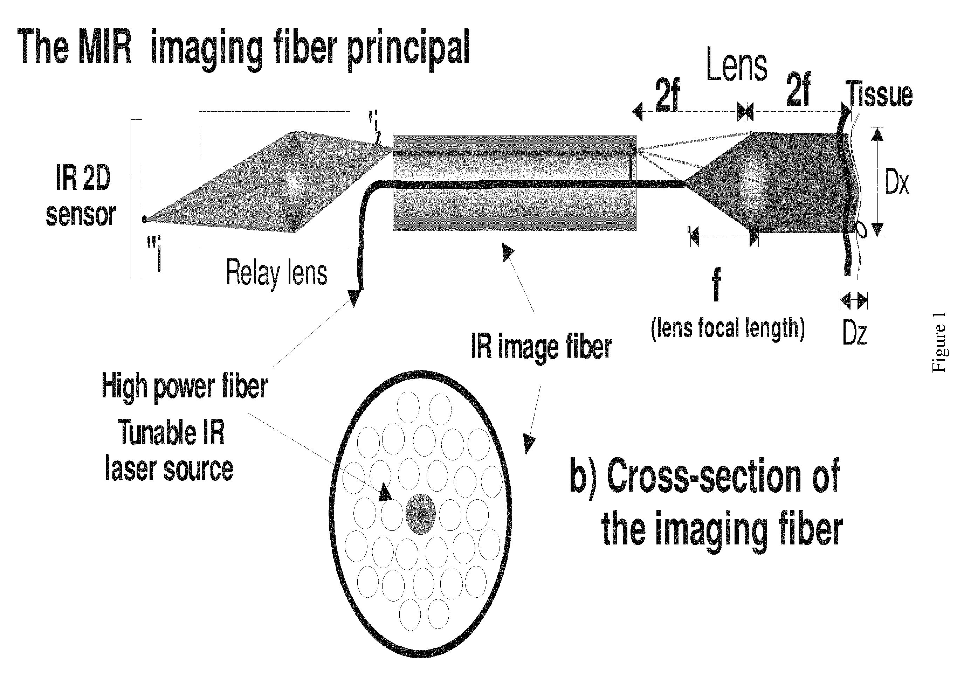

Figure 1 shows one embodiment of the imaging device of the invention. The imaging device comprises a mid infrared, MIR, light source, at least one optical fibre adapted to transport MIR light emitted from the MIR light source, an imaging probe coupled to the optical fibre and adapted to provide a MIR light beam to an imaging area, and optics adapted to control the size of the MIR light beam for imaging.

The MIR light source comprises a high repetition rate (>100 MHz) picosecond pulsed laser source with matching Optical Parametric Oscillator (OPO) with a tunable wavelength in the MIR (λ=2.5-20 µm). The brightness of the infrared laser source is 2 to 4 orders of magnitude higher than that of a synchrotron and 5 to 7 orders of magnitude higher than that of a thermal source. This high intensity laser enables spectro-microscopic imaging with high pixel numbers and spatial resolution that makes *in vivo* MIR imaging compatible with conventional standards of visible imaging (e.g. with 1000x1000 pixels).

The optical fibre probe provides a MIR beam spot, the size of which can be equivalent (i.e. approaching ∼λ) or lower (i.e. <λ or more preferably <λ/2) than the excitation wavelength of the laser. The optics at both the proximal and distal end of the device are conditioning, modulation and patterning optics which control the final size of the imaging spot, both for large area imaging at video rate or higher, and smaller area imaging at high spatial resolution (e.g. sub wavelength, <λ or super-resolution, <λ/2);

The MIR optical fibre serves as a conduit for both MIR excitation and image signal transport. In one embodiment of the invention, a bundle of such thin MIR optical fibres is used, with one optical fibre dedicated for excitation while the other fibres are used for image signal collection. The number of such image fibres depends on the number of pixels of images that can be collected at a time. Access to the disease site can be obtained through minimally invasive devices such as catheters and fine access needles. The imaging site may include tissues, cells, sub cellular components, enzymes, proteins, and amino acids.

In another embodiment, the core MIR fibre is surrounded by a bundle of thin fibres that are capable of transporting visible and near IR (NIR) light. In this case, the MIR image signals are transported by these vis-NIR optical fibres through a conversion of MIR light into vis-NIR lights, and the vis-NIR image can be formed using a charged coupled device (CCD) camera. Some of these thin vis-NIR fibres can also be used for rapid visualisation of the site of interest prior to MIR imaging. Such an arrangement also enables the integration of multi-modality, such as those involving visible light in spectroscopy, such as Raman, CARS and SFG, with the modality of MIR imaging. For MIR imaging at high spatial resolution, special configurations such as attenuated total reflection (ATR), special lens (Si, ZnS or ZnSe), optical patterning (using phase plate or a deformable mirror) are required at the distal end of the imaging device. These configurations are discussed below in more detail.

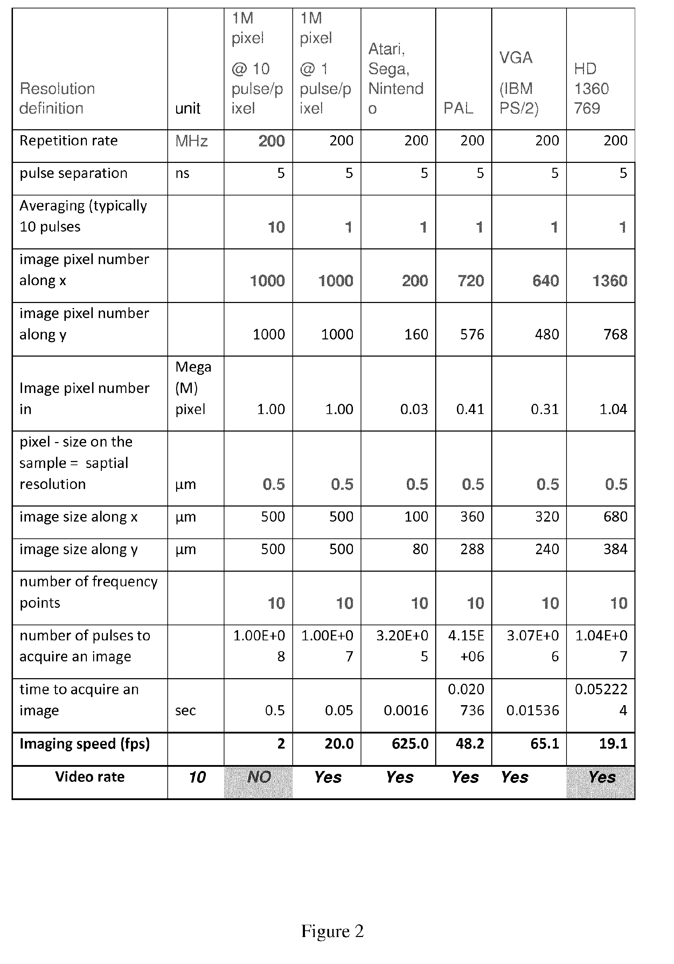

Figure 2 shows the imaging speed of the MIR device using a 200 MHz pulse. This figure illustrates how the speed of acquisition can vary with the repetition rate of the pulsed laser. The frame per second (fps) has been estimated assuming a pulse separation of over 5 ns for the response time of the infrared detector and accumulative thermal effects. The compatibility of the imaging speed with current video imaging standards is also shown.

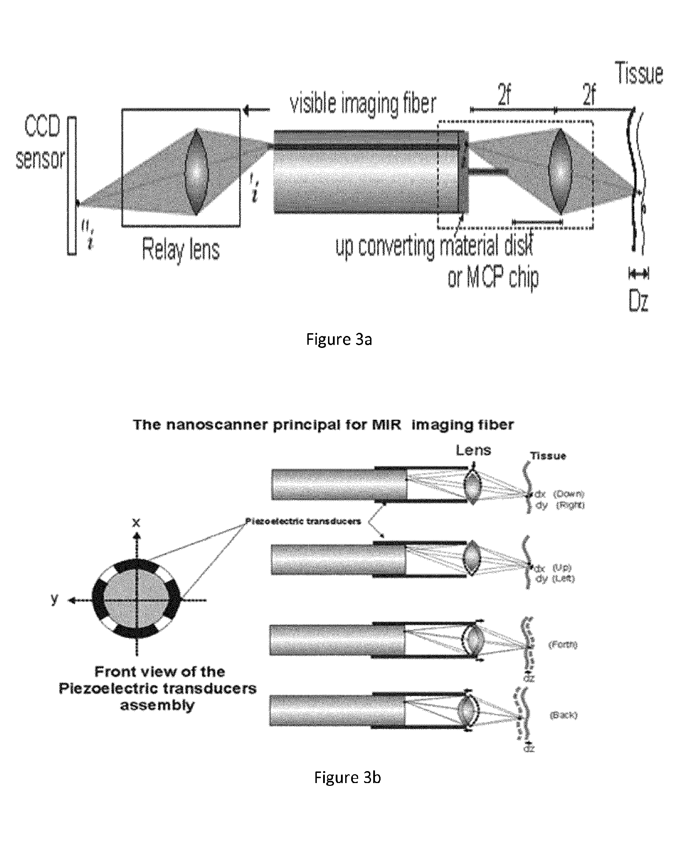

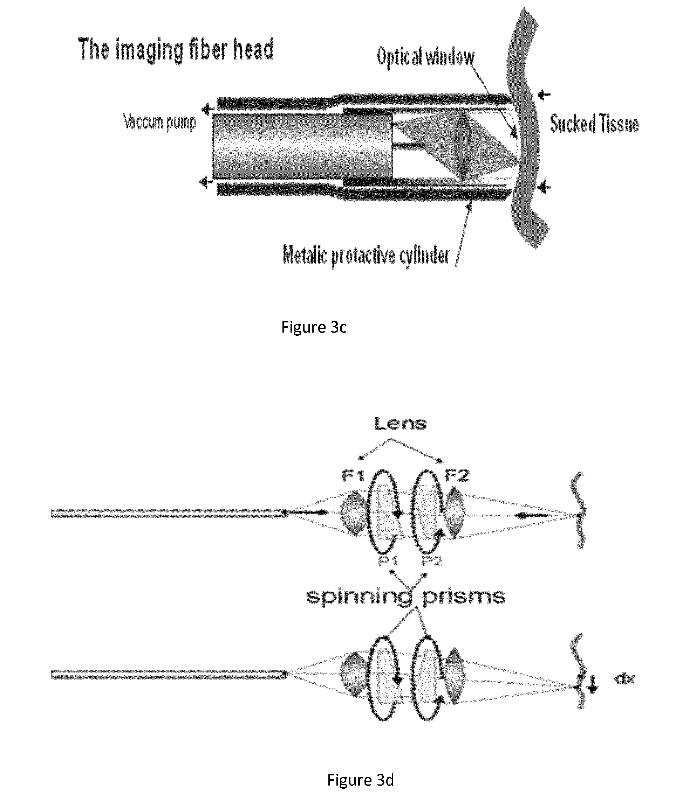

Figure 3 shows a number of configurations of MIR imaging provided by the device of the present invention, in an embodiment when a CaF2 or ZnS based MIR lens or zooming optics are used. The MIR illumination of the imaging area is achieved by transporting the MIR beam from the laser at the proximal end to the distal end of the device through the transporting MIR optical fibre, as shown in Figure 3a. The optical fibre is positioned at the focal point to ensure the best possible homogenous illumination *in vivo.* The optical fibre may comprise polycrystalline silver halides capable of transmitting MIR of between 2 and 17 µm wavelength.

In a point by point spectroscopic imaging used in endoscopy, the optical probe or the focused beam requires movement. This can be achieved by suitable scanner systems. One such system is a nanoscanner piezoelectric system, which enables both 2D and 3D imaging at sub-wavelength and super resolution, as shown in Figure 3b. In many cases such scanning can be facilitated by immobilizing the investigation area of the tissue by using suction, as shown in Figure 3c. Such immobilised sections can then be imaged using both small-field of view, nanoscale scanning and large field of scanning, for example by using a spinning prism, as shown in Figure 3d. A miniature suction system permits a convenient way to realise MIR endoscopic imaging in reflection, and enables very high spatial resolution to be achieved in MIR.

The MIR fibres can be of any suitable number and size depending on the application. A bundle of large MIR imaging fibers will ensure fast imaging. However, the size of such bundles can be a limiting factor especially when accessing anatomical sites through minimally invasive procedures. A bundle of small MIR imaging fibers could alternatively be used, and rotated/moved using a scanner system. A single MIR imaging fiber along with a scanner system is suitable for high resolution imaging, but at a slower imaging speed. A gradient index (GRIN) fibre, which is short and can be mounted at the distal tip of the MIR excitation fibre, could also be used.

The image signals may be transported from the distal end to the proximal end of the device using either MIR optical fibers only, using visible or NIR fibres, or using a combination of visible, NIR and MIR fibres. A bundle comprising a combination of MIR, NIR and visible optical fibres are advantageous to enable both large and small field of view imaging modes by using two different wavelengths. One of them, for example, can be used to use NIR excitation at lambda=∼ 1 um, which corresponds to the maximum transmission of a NIR beam through the surrounding medium (e.g. blood and water), and can be used for a large view inside the organ. MIR excitation can then be used for chemical imaging at high resolution over a selected and targeted relatively smaller region of tissue, for biopsy or thermal resonant therapy. Selecting the wavelength around 1 µm also has the advantage of the possibility of integrating a miniaturised CCD camera inside the probe head.

In one embodiment, the MIR image signal can be up-converted into visible or NIR light after image collection through a MIR lens. Up conversion can be achieved using night-vision techniques, such as by using a multi-channel plate, MCP (as shown in Figure 3a), or by means of a suitable up conversion material. The use of standard visible light optical fibres has a number of advantages. Firstly, it enables the fibre assembly to be of a much smaller size, and thus compatible with endoscopic access devices such as catheters and needles. The use of visible fibers also allows higher image resolution, by allowing the use of numerous micron size range visible fibres. It also allows for the use of the high imaging speed of the CMOS camera and the high detection sensitivity of state of the art 2D detectors, such as an electron magnified camera (sensitive to a few photons).

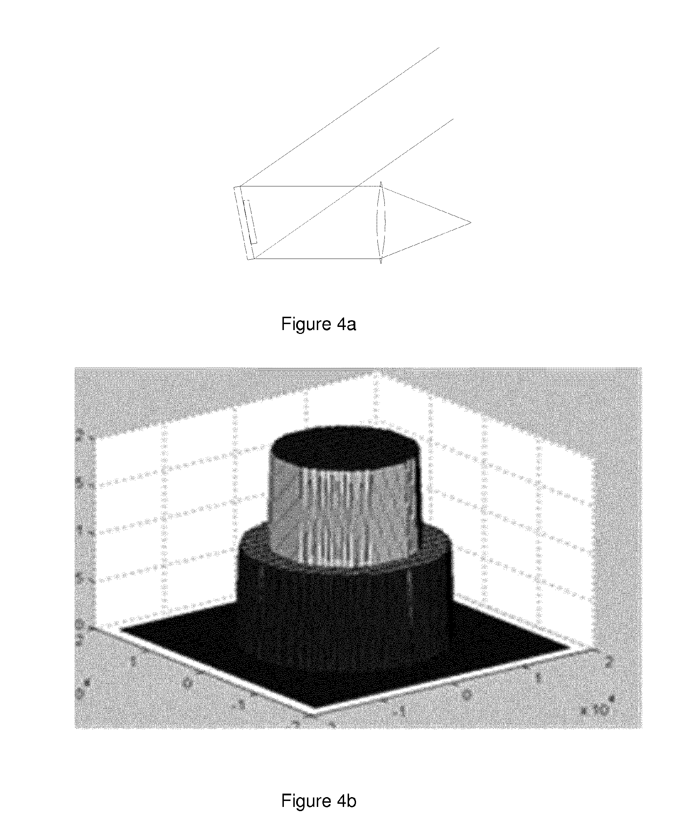

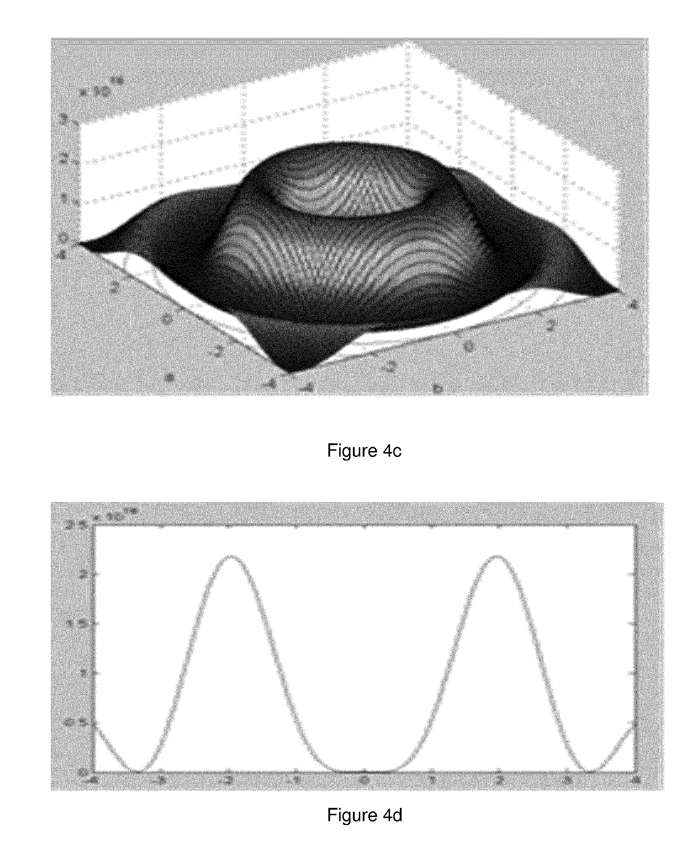

In order for the device of the present invention to achieving super resolution, optical patterning is required. This can be done by a number of different techniques. In one embodiment, a phase plate is used to create a doughnut optical pattern. An alternative method of creating a doughnut optical pattern is to use a deformable mirror with a symmetric deformation. This can be achieved by taking a circular mirror and inducing a circular deformation in its centre, and then by controlling the depth, and the diameter of deformation at the zero intensity at the centre of the beam can be induced at the focal point. Figure 4a illustrates such patterning of a homogenous beam by illuminating a 24 mm diameter mirror at a focal length of 12 mm using a 3.4 µm MIR excitation. Figure 4a illustrates the experimental configuration, Figure 4b illustrates the symmetric mirror shape (the x axis is in µm), while Figure 4c illustrates the intensity at the focal plane and Figure 4d illustrates the line profile across the doughnut.

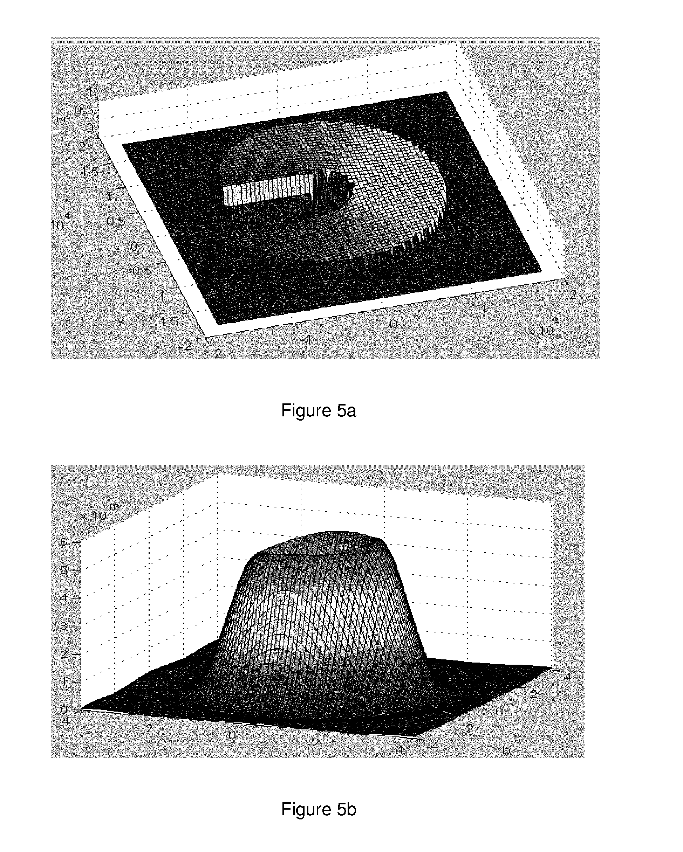

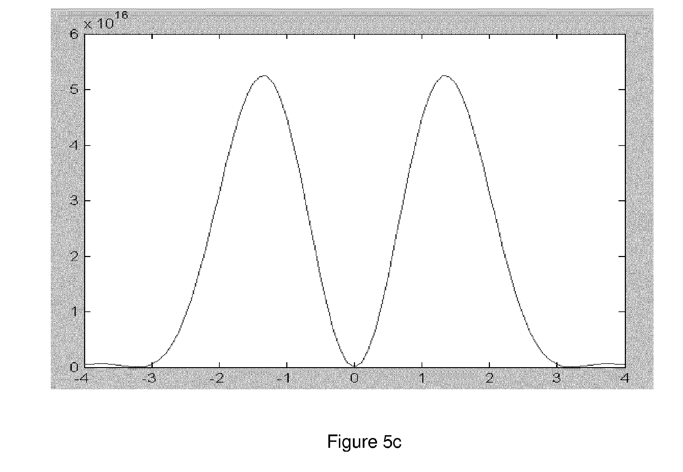

This method can be further improved through the use of a helicoidal mirror. A helicoidal mirror can be created by taking a flat metallic mirror and cutting in it a correct shape by electro-erosion, and then applying a tension on both sides of the step. The effect of the angle of incidence on the mirror and the effect of the size of the centre hole are small on the quality of the doughnut optical pattern. The main advantage of such mirrors over a phase plate is that with such mirrors ∼90% transmission is possible. Most importantly, these mirrors offer frequency tunability over the full infrared and visible range of spectra. This is currently extremely difficult to achieve using phase plates. In this case, the doughnut profile is the same as the ideal profile that can be achieved using a phase plate. Figure 5 illustrates simulation results for such mirrors (the x axis is in µm). Figure 5a shows a helicoidal mirror shape creating the doughnut pattern, while Figure 5b shows the intensity profile at the focal plane, and Figure 5c shows the line profile across the doughnut.

In addition to the far field approach, the device of the present invention can also provide super resolution in MIR using a near field approach. In one embodiment this is achieved using the standing evanescent waves generated by Attenuated Total Reflection (ATR) configuration. Experimental implementation of this scheme is relatively straightforward in the case of endo-microscopy, as such a scheme doesn't need complex beam patterning, as would be in the generation of doughnut profile, nor does it require the sample to be scanned for point spectroscopy. A spatial resolution in the range of ∼lambda/35 is possible. With a Si hemisphere in the optical fibre probe, a resolution of 600 nm is possible in ATR configuration with 3.4 µm even without implementing a pump/probe scheme. This sub-wavelength resolution imaging can be carried out at the video rate.

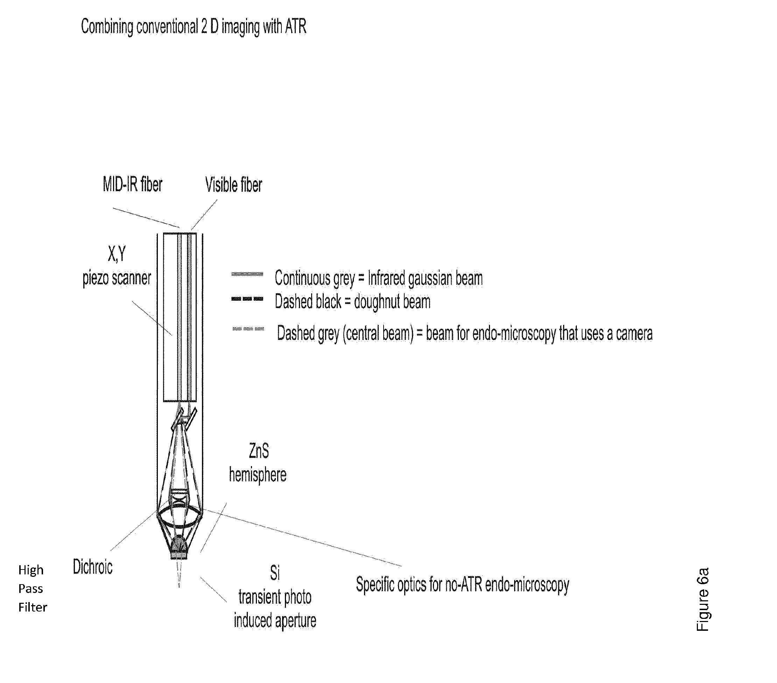

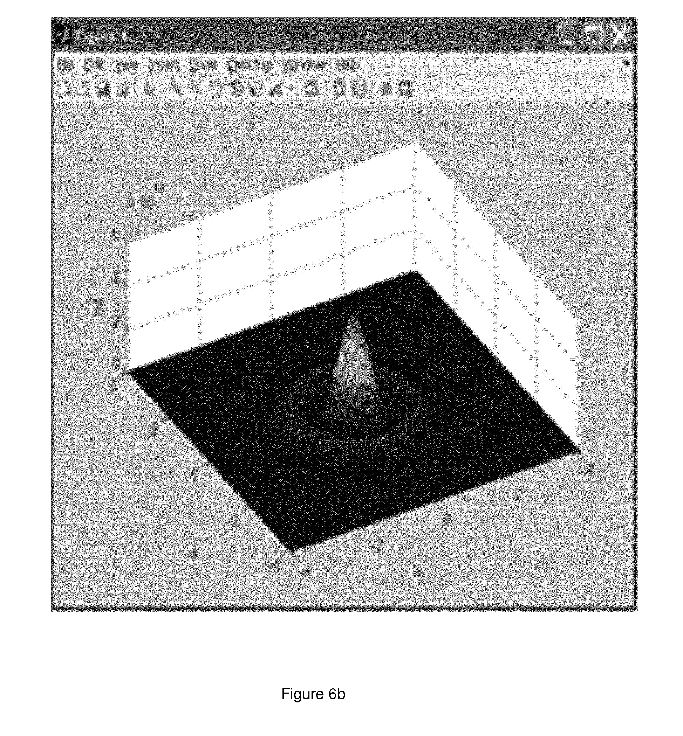

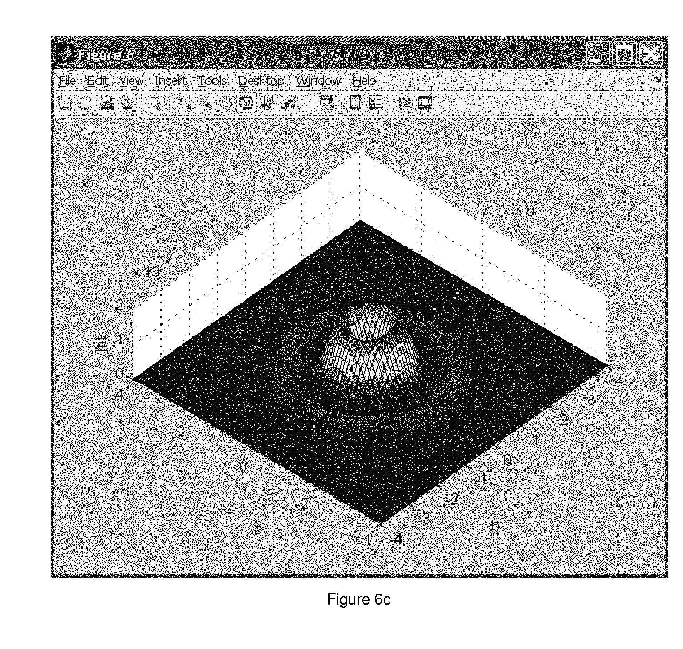

In yet another embodiment a photoinduced aperture scheme is used to provide super resolution, as outlined in Figure 6. Here the distal end of the device of Figure 6a is provided with two different fibers: one for MIR (shown in Figure 6b) and the other for a visible light doughnut beam (shown in Figure 6c). The photo induced aperture is created using a silicon/germanium film plate (or a film) deposited on the flat face of a ZnS or ZnSe hemisphere. A doughnut pulse will excite electrons in the conduction band of the Silicon, which entails transient reflectivity, except at the doughnut centre. A Gaussian MIR pulse sent in sequence interacts with the molecules in contact with the hemisphere only where silicon is not reflecting, for example in the centre of the doughnut. As there is no requirement for saturation, an infrared vibration MIR pulse with much lower intensity can be used in this embodiment. This may be advantageous for in vivo imaging where the damage threshold of tissues can be low that may hinder a conventional saturation based pump/probe super-resolution MIR imaging scheme. The ability to use a visible pump doughnut is also advantageous, as a smaller size doughnut can be made that will allow easy separation of a pump and a probe at a detector. A ZnSe hemisphere coupled with a photoinduced aperture in Si can provide a resolution below 100 nm in ATR configuration at a 3.4 µm excitation wavelength. The pulse energy to generate the photo induced aperture is on the order of 1 µJ, which can be obtained at kHz repetition rate of the laser. This will however slow down the imaging, so video rate megapixel imaging will not be possible for such imaging. Importantly, the MIR excitation fibre itself can function as a photo-induced pinhole to enable confocal, 3D imaging. It should be noted that in the embodiment of Figure 6a, the dichroic/high pass filter allows transmission of the central beam for 2D non-ATR imaging similar to an endo-microscopy set up that uses a camera configuration, blocks MIR for ATR configuration and blocks the specific wavelength for photo induced aperture laying between 400nm and 1064nm. For the non-ATR imaging, an appropriate wavelength must be chosen which is transmitted by the hemisphere assembly. For example, for a Ge or Si hemisphere, the beam must be in the NIR range (such as for example 2 µm). For a ZnS or a diamond hemisphere without Si coating, a visible light can be used. On the other hand, when a Si coating is applied to the ZnS or to the diamond hemisphere, the beam must be chosen from the NIR range.

For attenuation during imaging one needs to choose an appropriate wavelength which is transmitted by the hemisphere/coating assembly. For example, For a Ge or Si hemisphere, the beam must be in the NIR range, e.g. 2 micron wavelength, while for a ZnS or diamond hemisphere without any Si coating, a visible light can be used. On the other hand, when a Si coating is applied on the ZnS or the diamond hemisphere, the beam must be chosen from the NIR range. Sub-λ imaging does not need optical patterning, and is relatively straightforward to implement using a micro-lens with higher refractive indices. It combines a high resolution, large investigation area, and depth information with an unprecedented imaging speed (>10 fps) for MIR. With a >100 MHz repetition rate, a ∼ 700 nm resolution (∼lambda/5) with 10 µm depth is possible. Based on the estimates given in Figure 2, megapixel images of a 5 µm pixel size over a 0.5x0.5 cm² area can be obtained at a video rate (>10 fps) leading to 4D MIR imaging.

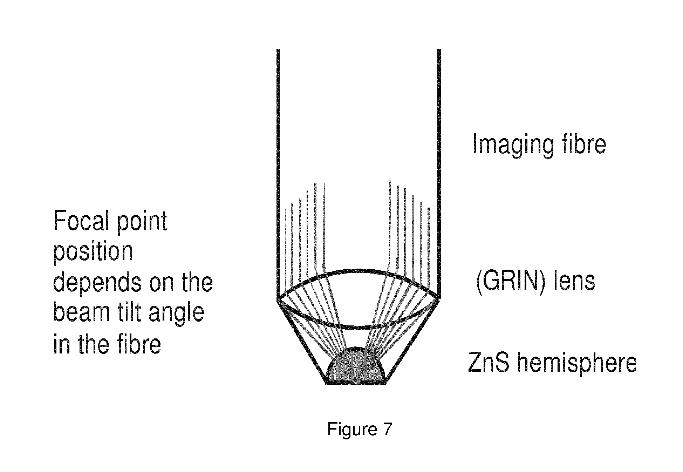

Figure 7 illustrates an embodiment of the device for use in 2-D infrared microscopy in ATR with single mode MIR optical fibres for diffraction limited resolution imaging. The spatial resolution is improved by a factor ~2 with respect to conventional infrared microscopes because of the refractive index of the hemisphere material. If the endoscope is 600 µm large, the surface which will be imaged will can be about 200-400 µm in diameter. This is compatible with the minimum size of the state of the art MIR optical fibres made of polycrystalline silver halide. This design can be upgraded to super resolution by sending a sequence of a doughnut and Gaussian beams through the fibre as explained above, or by generating a pump standing wave in the ZnS hemisphere. For such endoscopic MIR imaging, only reflection mode of imaging is possible.

The external part of the hemisphere, as shown in Figures 6 and 7, is used for ATR microscopy, and the central part, is used for conventional 2D imaging. This arrangement is practical and advantageous for a surgeon, as it allows the surgeon to 'see' conventional optical images during the manoeuvring of the distal end of the endoscope, while at the same time allowing the surgeon to bring the distal end in contact with the 'targeted' tissue/anatomical site for ATR-MIR imaging.

It has been found that the utilisation of single-mode fibers (SMFs) in the device of the present invention is advantageous over multi-modal fibres (MMFs). This is due to cost-effectiveness, and the ability of a complete elimination of multipath/intermodal dispersive effects. However, material and waveguide dispersion is still present in SMFs, with the extent of this depending on the source linewidth, and thus will restrict the ultimate spatial resolution that can be achieved.

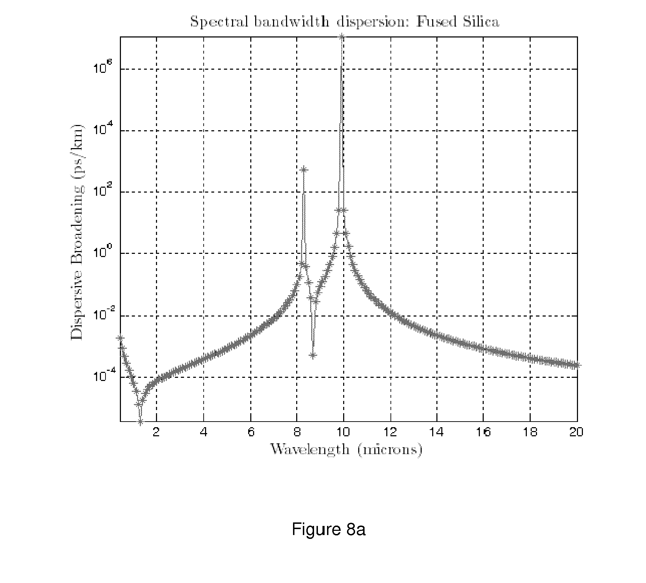

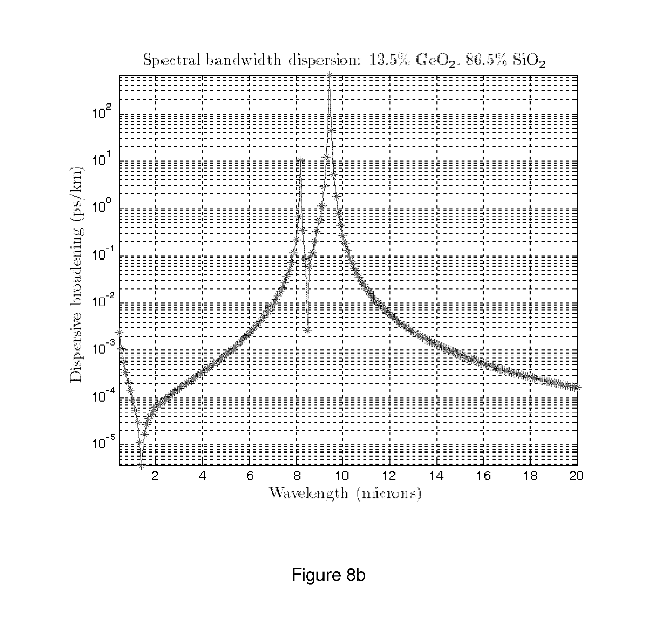

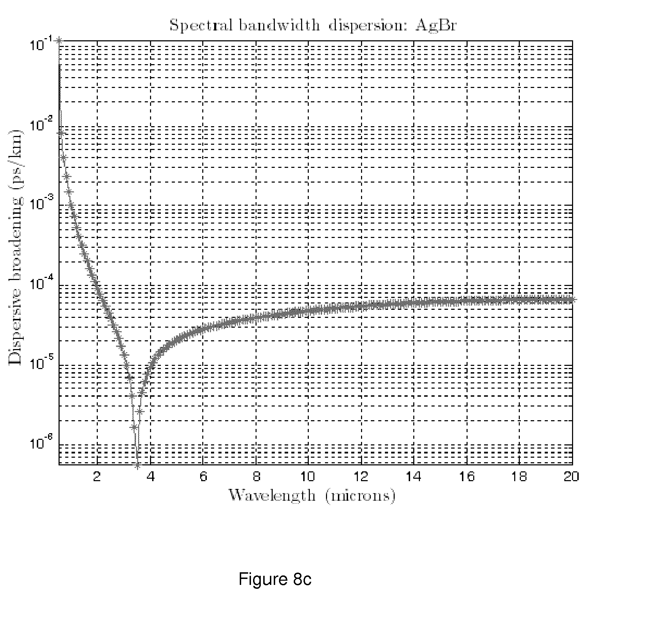

In the differential mode of MIR imaging for superresolution, the difference in pump-probe signal is used when the pump spatial pattern is changed between 'nodal' (through a vortex phase plate) and 'anti-nodal' beam schemes (through an unmodified Gaussian MIR laser pulse). The effect of pulse width broadening during the propagation of a simple Gaussian distribution through a dispersive material has been modelled computationally in Figure 8 to quantify the effect of the distortion of the Point Spread Function (PSF). Figure 8 shows graphs of simulated spectral dispersion for a wavelength range of 0.4 - 20 µm for (a) fused silica (b) SiO2 doped with 13.5% GeO2 and (c) AgBr fibers, each employing a FWHM spectral pulse linewidth of 1.6 µm over a fiber propagating length of 1 m. It can be seen from these graphs that there is a clear advantage of using a silver halide based optical fibre, as the dispersion broadening is significantly less over the most of the spectral region for this material.

Many biomarkers show little or subtle differences between their healthy and pathological states in their MIR spectra even in the fingerprint regions. It is known in the art that chemical differences can be convoluted under a broad envelop of a characteristic vibrational transition (peak), and the second derivative of the spectra with respect to the wavelength often is used as an aid to distinguish between these apprently similar spectra. However, due to the monochromatic nature of the MIR laser pulses used in endoscopic imaging, operating a second derivative of a spectra with respect to the wavelength is difficult. In one embodiment of the invention this is achieved by modulating the wavelength output from the OPO or OPA around the wavelength of interest, such as for example by placing a non linear crystal or other optical element of the OPO OPA with a piezo element that can modulate the laser wavelength. For small oscillations and a lockin amplification in the 'locked' state onto the modulation of the piezo element will provide the first derivative of the spectra at the measured wavelength (ie the central wavelength in the modulation), while the 2nd harmonic on the lockin will give the second derivative. Imaging using this second harmonic will provide a second harmonic image, which can then be utilised for disease diagnosis.

In addition to the minimally invasive endo-spectro-microscopy, the device of present invention can be used in non-invasive *in vivo* imaging and *ex vivo* imaging. For example, blood is opaque to visible light, which thus requires a replacement of blood with clear saline solution during angioscopy and sufficient power to overcome absorption. Optical scattering in blood remains unaffected by any increase in the intensity of visible light. As cells typically have dimensions in the region of 1-20 µm, illumination by IR light will decrease the scattering inversely as the square of the wavelength. This means that scattering in blood and other opaque body-fluid environments will be improved dramatically by using MIR wavelengths (2-20 µm) which are comparable to particle dimensions. Additionally, only certain IR wavelength regions are semitransparent to water and haemoglobin. As such, certain wavelength bands satisfy the dual criteria of low scattering and low absorption: namely 1.4-1.8 µm, 2.1-2.4 µm, 3.7-4.3 µm, 4.6-5.4 µm and 7-14 µm, meaning that cellular imaging with high resolution and high sensitivity in the blood stream is feasible. The longer wavelength regions (>3.7 µm) require higher intensity light sources, due to the increase in water absorption in these regions. IR absorption by water can be minimised in a tuneable laser by simply choosing to work in a separate window and/or using filters to block frequencies where water absorbs IR. Access to the blood stream can be obtained exogenously through the skin, or through fine or conventional needle techniques.

While the device of the present invention has been described for use with MIR in vivo imaging, the device can also be used for visible and near IR (NIR) visualisation, as well as other spectroscopic imaging such as those based on Raman spectroscopy, Sum Frequency Generation (SFG) and Coherent Anti-Stokes Raman Spectroscopy (CARS).

The device of the present invention has numerous advantages. It enables the detection of diseases through creating fast images of biomarkers over multiple length scales, ranging from a 0.5 centimetre to around 0.5 micron. The fact that the device may be integrated with biopsy and aspiration devices enables video rate chemical biopsy that can detect diseases rapidly and with high certainty, with insignificant photoionization damage or mutation of biomolecules and tissues. The image acquisition at high speed enables such imaging at a video rate preferable and interpretable by medical professionals.

The device of the present invention also provides a low optical dispersion for a transmitted short laser pulse in the range of sub-picosecond, as well as low nonlinear phenomena. Furthermore, the device allows variable spot sizes, ranging from a less than a micron (super resolution) to tens of micron, tunable to the size of the tissue area needed to be imaged at the shortest possible time. The device may also be used with both MIR and visible fibers, as well as single and multi fibre assemblies. In addition, the device enables minimally invasive imaging for almost any anatomical imaging area. The small size of the device is also very suitable for imaging applications.

The use of an IR laser also means that a simple targeted therapy by laser ablation of the local chemical aberration is also possible, so that the device may not only diagnose diseases early, but also treat them early. The highly selective absorption at characteristic wavelengths allows a "search and destroy" procedure to locate pathological molecules or tissue parts and the dissociation of that particular molecule or tissue part only.

The embodiments in the invention described with reference to the drawings comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a floppy disk 10 or hard disk. The carrier may be an electrical or optical signal which may be transmitted via an electrical or an optical cable or by radio or other means.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. An endoscopic mid infrared in vivo imaging device comprising:
a mid infrared, MIR, light source;
at least one optical fibre adapted to transport MIR light emitted from the MIR light source;
an imaging probe coupled to the optical fibre, wherein the imaging probe is adapted to provide a MIR light beam to an imaging area; and
optics adapted to control the size of the MIR light beam for imaging.

2. The device of Claim 1, wherein the MIR light source comprises a laser integrated with a matching optical parametric oscillator, OPO, wherein the laser is adapted to generate picosecond infrared pulses at a high repetition rate with a tuneable wavelength.

3. The device of Claim 2, wherein the pulse repetition rate is greater than 30 MHz.

4. The device of Claim 3, where the pulse repetition rate is greater than 100 MHz.

5. The device of any of Claims 2 to 4, wherein the wavelength of the laser is tuneable between 2.5 and 20 microns.

6. The device of any of the preceding claims, wherein the size of the MIR light beam is equivalent to or lower than the wavelength of the MIR light source.

7. The device of any of the preceding claims, wherein the device comprises a proximal end and a distal end, wherein the MIR light source is provided at the proximal end, and wherein the optics are provided at both the distal end and at the proximal end.

8. The device of Claim 7, wherein the at least one optical fibre comprises one optical fibre adapted to transport MIR light emitted from the MIR light source from the proximal end to the distal end and one or more other optical fibres.

9. The device of Claim 8, wherein the one or more other optical fibres are adapted to transport near infrared or visible light.

10. the device of Claim 8 or Claim 9, wherein the one or more other optical fibres are adapted to transport images collected from the imaging area from the distal end to the proximal end of the device.

11. The device of any of the preceding claims, wherein the at least one optical fibre comprises polycrystalline silver halide optical fibres.

12. The device of any of the preceding claims, wherein the optics are adapted to control the size of the MIR light beam to provide large area imaging at the video rate or higher.

13. The device of any of Claims 7 to 12, wherein the optics comprise a lens or zooming optics at the distal end of the device and a relay lens at the proximal end of the device.

14. The device of Claim 13, wherein the optics comprise a CaF2 or ZnS or gradient index lens or zooming optics at the distal end of the device.

15. The device of any of Claims 7 to 13, wherein the optics at the distal end of the device are adapted to provide high spatial resolution imaging.
